# EUROPEAN PATENT SPECIFICATION

(11) **EP 0 536 302 B1**
(45) Date of publication and mention of the grant of the patent: **27.08.1997**
(21) Application number: 91913213.4
(22) Date of filing: 27.06.1991
(51) Int. Cl.: A61K 39/42, A61K 39/40, A61K 39/00, A61K 39/02, C07K 14/00

(54) **IMPROVED ADJUVANTS AND VACCINES**
VERBESSERTE ADJUVANTIEN UND IMPFSTOFFE
ADJUVANTS ET VACCINS AMELIORES

(30) Priority: 27.06.1990 US 544831; 21.06.1991 US 716807
(43) Date of publication of application: 14.04.1993
(73) Proprietor: EMORY UNIVERSITY, Atlanta, GA 30322 (US)
(72) Inventor: HUNTER, Robert, L., Tucker, GA 30084 (US); TAKAYAMA, Kuni, K., Madison, WI 53705 (US)
(74) Representative: Fleischer, Holm Herbert, Dr.
(86) International application number: US9104716
(87) International publication number: WO9200101

(56) References cited:
- US-A- 4 606 918
- US-A- 4 609 546
- US-A- 4 770 874
- US-A- 4 772 466
- INFECTION & IMMUNITY, vol. 56, no. 5, May 1988; ZIGTERMAN et al., pp. 1391-- 1393/
- JOURNAL OF IMMUNOLOGY, vol. 127, no. 3, September 1981; HUNTER et al., pp. 1244-1250/
- JOURNAL OF IMMUNOLOGY, vol. 133, no. 6, December 1984; HUNTER et al., pp. 3167- 3174/
- JOURNAL OF BACTERIOLOGY, vol. 155, no. 1, July 1983; STRITTMATTER et al., pp. 153-158/
- INFECTION & IMMUNITY, vol. 57, no. 4, April 1989; TAKAYAMA et al., pp. 1336- 1338/
- IMMUNOLOGY, vol. 65, 1988; ZIGTERMAN et al., pp. 37-42/
- INFECTION & IMMUNITY, vol. 58, no. 11, November 1990; LOPPNOW et al., pp. 3743- 3750/
- INFECTION & IMMUNITY, vol. 59, no. 1, January 1991; KIRKLAND et al., pp. 131-136/
- ABSTRACTS of the 90th Annual Meeting of the American Society for Microbiology, May 1990; KALISH et al., p. 130, no. E-65/
- "Copolymer Adjuvants and Titermax" by Robert L. Hunter, Margaret R. Olsen and Beth Bennett, in "The Theory and Practical Application of Adjuvants", 1995, eds. D. E. S. Stewart-Tull, John Wiley & Sons Ltd.

## Description

### Technical Field

The present invention relates to vaccine adjuvants and to improved vaccines that use the adjuvants. The adjuvants can be designed so that the immune response is predominantly antibodies of a desired isotype; *e.g*., IgG2 or IgG3 isotypes in mice or the corresponding isotypes in man and other animals, thereby improving protection by a vaccine. In addition, the improved vaccine and adjuvant of the present invention provides long lasting protection.

### Background Art

The term "antigen" is defined as anything that can serve as a target for an immune response. The immune response can be either cellular or humoral. The term "vaccine" is defined herein as a suspension or solution of antigenic moieties, usually consisting of infectious agents, or some part of the infectious agents, that is injected into the body to produce active immunity. The antigenic moiety making up the vaccine can be either a microorganism or a natural product purified from a microorganism, a synthetic product or a genetically engineered protein, peptide, polysaccharide or similar product. The term "cell mediated immunity" is defined as an immune response mediated by cells rather than by antibody. It includes, but is not limited to, delayed type hypersensitivity and cytotoxic T cells. The term "adjuvant" as used herein is any substance whose admixture with an injected immunogen increases or otherwise modifies the immune response. A "hapten" is defined herein as a substance that reacts selectively with appropriate antibodies or T cells but the hapten itself is usually not immunogenic. Most haptens are small molecules or small parts of large molecules, but some macromolecules can also function as haptens. The term "conjugation" is defined herein as the covalent or other form of linking two or more molecules. It can be accomplished either by chemical means or *in vivo* by biologic means such as genetic engineering. The term "isotype" is a subtype of an antibody. The term "lipopolysaccharide" (LPS) is a amphipathic glycophospholipid obtained from the outer membrane of gram-negative bacteria which has a hydrophobic moiety called lipid A and a sugar moiety (polysaccharide or oligosaccharide). The term "non-toxic LPS" is defined as an LPS with very low toxicity based on one or more measurements of 50% lethal dose in animals (LD₅₀), 50% chick embryo lethal dose (CELD₅₀), pyrogenicity in rabbit, or dermal Shwartzman reaction. The *in vitro* measurements of the induction of either/both tissue necrosis factor and IL-1 by macrophage can also be used to determine the toxicity of LPS. The term "detoxified LPS" is defined as being LPS with reduced toxicity due to chemical modification of the structure of Lipid A moiety, *i.e*., removal of one phosphate group, removal of one to three fatty acyl groups, the introduction of new functional groups (e.g., methyl, acetyl, alcohol and the like), or partial reduction or oxidation.

An effective vaccine must induce an appropriate response to the correct antigen or antigens. There are several distinct types of immune responses which vary in their ability to confer protection against particular diseases. For example, antibodies may confer protection against bacterial infections, but cell mediated immunity is required for eliminating from the body many viral infections and tumors. There are multiple distinct types of antibody and cell-mediated immune responses. Cell-mediated responses are divided into two basic groups: 1) delayed-type hypersensitivity in which T cells act indirectly via macrophages and other cells or cell products, and 2) cytotoxicity in which specialized T-cells specifically and directly attack and kill infected cells.

There are five major classes of antibody: IgM, IgG, IgE, IgA and IgD. These classes have distinct functions in the immune response. IgG, the dominant class in the blood, is subdivided into several different subclasses or isotypes. In mice, these isotypes are IgG1, IgG2a, IgG2b, and IgG3. In humans, the isotypes are IgG1, IgG2, IgG3 and IgG4.¹ Similar isotypes have been defined in most other mammalian species in which they have been investigated. The nomenclature of IgG isotypes is different in different species because the names were coined before the structure or function of the antibody isotypes were understood. Although much still remains to be learned, the IgG isotypes appear to be highly conserved among mammalian species.

The IgG isotypes differ in their ability to confer protection to particular infections. IgG2a and IgG2b in mice activate complement, mediate antibody mediated cell mediated cytotoxicity and other functions. They are particularly effective in conferring protection against many bacterial, viral and parasitic infections. The counterparts in humans appear to be IgG1 and IgG3. In contrast, murine IgG3 is particularly effective in conferring protection against bacteria with polysaccharide coats such as the pneumococcus. The human counterpart seems to be IgG4. Isotypes such as IgG1 in mice do not fix complement, neutralize toxins effectively, but are markedly less effective for many bacterial and viral infections. Because the different IgG isotypes differ markedly in their ability to confer immunity, it is important that vaccines induce the most appropriate isotype for a particular infection. Even though the nomenclature is different, available evidence and modern theory indicate that the properties of immunogens which determine the isotype of antibody produced are similar across mammalian species. In other words, an immunogen which stimulates delayed type hypersensitivity or complement fixing IgG antibody in one species will generally stimulate similar responses in other species.

Biosynthetic and recombinant DNA technology is permitting development of vaccines possessing antigenic epitopes that were previously impossible to produce. Current vaccine candidates include virtually all infectious agents, allergens and even host components such as hormones and molecules involved in autoimmune diseases, cancer and other diseases. The infections agents include, but are not limited to, viruses, bacteria, parasites, rickettsiae and fungi. Hormones are being evaluated as vaccines for diverse purposes such as prevention of pregnancy and treatment of disease. Vaccines for treatment of cancers, such as melanoma, are being evaluated in animals and man. In each case, optimal effect of the vaccine depends upon stimulating the appropriate type, intensity and duration of the immune response.

The work on the parasitic disease malaria is especially important. This disease affects in excess of 200 million people per year worldwide and is the most important disease in the world in terms of morbidity and loss of work. The techniques of genetic engineering have been used to identify, and now to produce in substantial quantities, several peptides and proteins associated with malarial parasites. In particular, a twelve amino acid peptide from the sporozoite stage has been determined to carry an important antigenic site. Antibodies against this particular peptide can kill the parasite immediately after it is injected. Unfortunately, this peptide, by itself, does not produce an adequate immune response. Each species of malaria has a different peptide, but the characteristic structure and repeat units is found in all of them.

In an effort to induce an effective immune response to the sporozoite peptide, the peptide has been conjugated with carriers and administered with adjuvants. To date, however, the adjuvants used with the peptide or peptide conjugates have not produced satisfactory results. Similarly important antigens have been identified on the blood stages of malarial parasites, but available vaccine formulations have been unable to induce protective immunity.

Human immunodeficiency virus (HIV) causes AIDS. Many recombinant and peptide antigens have been prepared from HIV. There is evidence that antibodies against these antigens can neutralize the virus and that the body's immune response is able to prevent or control infections. However, generally effective vaccines to induce protective immune responses against HIV have remained an elusive goal. *Hemophilus influenza* and *pneumococcal pneumonia* provide further examples. The important antigens of these bacteria are polysaccharides which elicit protective immune responses poorly in infants and elderly persons who are in most danger from these infections. Similar situations exist for numerous other viral, bacterial and parasitic infections in addition to tumors and other diseases which can be modulated by immune responses. Modern science has provided the means to identify and produce antigens from most conditions which are influenced by immune responses. The failure of many new antigens to induce optimal protection has highlighted an increasing need for means to influence the type, intensity, and duration of immune response produced by vaccines.

Thus, interest has arisen in the development of potent, nontoxic adjuvants that will enhance, and perhaps more importantly, modulate the immunogenicity of haptenic epitopes. In addition, adjuvants are needed for use with conventional vaccines to elicit an earlier, more potent, or more prolonged response of the appropriate type. Such an adjuvat would also be useful in cases where antigen supply is limited or is costly to produce.

The development of adjuvants has, until recently, been empirical. An enormous number of compounds have been found to modulate the immune response. These compounds have been notably diverse in both substance and function, a fact that has complicated attempts to discover the unifying mechanisms of adjuvant action. The elucidation of these mechanisms has lagged behind recent advances in the understanding of the immune system.

This diversity of adjuvants has presented difficulties in their classification. Adjuvants are occasionally grouped according to their origin, be it mineral, bacterial, plant, synthetic, or host product. The first group under this classification are the mineral adjuvants, such as aluminum compounds. The first use of aluminum compounds as adjuvants was described in 1926. Since that time antigens precipitated with aluminum salts or antigens mixed with or adsorbed to performed aluminum compounds have been used extensively to augment immune responses in animals and humans. Aluminum compounds and similar adjuvants appear to work through the following mechanism. The aluminum physically binds to the antigen to form particles. These form a depot of antigen in tissue following injection. Excretion of the antigen is slowed, thus prolonging the time of interaction between the antigen and antigen-presenting cells such as macrophages or follicular-dendritic cells. In addition, immunocompetent cells are attracted to the area of injection and are activated. Aluminum particles have been demonstrated in regional lymph nodes of rabbits seven days following immunization, and it may be that another significant function is to direct antigen to T cell containing areas in the nodes themselves. Adjuvant potency has been shown to correlate with inflammation of the draining lymph nodes. While many studies have confirmed that antigens administered with aluminum salts led to increased humoral immunity, cell mediated immunity appears to be only slightly increased, as measured by delayed-type hypersensitivity. Aluminum hydroxide has also been described as activating the complement pathway. This mechanism may play a role in the local inflammatory response as well as immunoglobulin production and B cell memory.

Primarily because of their excellent record of safety, aluminum compounds are presently the only adjuvants used in humans. They are, however, not without problems. Aluminum containing vaccines occasionally cause local reactions. Although allergic manifestations are not usually a clinical problem, aluminum compounds have been also said to attract eosinophils to the area of injection via a T-cell-dependent mechanism, to induce an IgE response if injected after antigen priming, and to elicit a carrier-specific cell population with helper function for IgE response. In addition, aluminum-containing vaccines cannot be lyophilized, thus necessitating refrigerated transport and storage with the resulting risk of contamination.

Finally, and most importantly, aluminum compounds are not always successful in inducing sustained protection from disease. This is due, in part, to their inability to induce the most appropriate isotypes of antibody or the optimal type of cell-mediated immunity. Thus, while aluminum salts have been a sufficient adjuvant for strong immunogens that require only antibody responses to elicit protection, they are not effective when used with weak immunogens like synthetic peptides of malaria or for introducing cell-mediated immune responses or IgG isotype of the type required to fight infections.

Another large group of adjuvants are those of bacterial origin. Adjuvants with bacterial origins have recently been purified and synthesized (*e.g*. muramyl dipeptides, lipid A) and host mediators have been cloned (Interleukin 1 and 2), providing chemically characterized products for study. The last decade has brought significant progress in the chemical purification of three adjuvants of active components of bacterial origin: *Bordetella pertussis,* lipopolysaccharide and Freund's Complete Adjuvant (FCA).

*B. pertussis* is of interest due to its ability to modulate cell-mediated immunity through action on T-lymphocyte populations. For lipopolysaccharide and Freund's Complete Adjuvant, adjuvant active moieties have been identified and synthesized which permit study of structure-function relationships.

Lipopolysaccharide and its various derivatives, including lipid A, have been found to be powerful adjuvants in combination with liposomes or other lipid emulsions. It is not yet certain whether derivatives with sufficiently low toxicity for general use in humans can be produced. Freund's Complete Adjuvant is the standard in most experimental studies. However, it produces severe local and systemic inflammatory reactions which may be severe enough to cripple or kill the host. It cannot be used in humans and may be banned for use in animals.

Many other types of materials have been used at various times as adjuvants. They include plant products such as saponin, animal products such as chitin and numerous synthetic chemicals. The source of an adjuvant among these categories has not proved particularly useful in predicting its biological properties.

Adjuvants have also been categorized by their proposed mechanisms of action. This type of classification is necessarily somewhat arbitrary because most adjuvants appear to function by more than one mechanism. Adjuvants may act through antigen localization and delivery, or by direct effects on cells making up the immune system, such as macrophages and lymphocytes. Another mechanism by which adjuvants enhance the immune response is by creation of an antigen depot. This appears to contribute to the adjuvant activity of aluminum compounds, oil emulsions, liposomes, and synthetic polymers. The adjuvant activity of lipopolysaccharides and muramyl dipeptides appears to be mainly mediated through activation of the macrophage, whereas *B. pertussis* affects both macrophages and lymphocytes. Recent and speculative approaches to immunopotentiation, such as the utilization of monokines and lymphokines, and the manipulation of the antigen, carrier, and adjuvant to augment the immune response are currently fashionable.

Small immunogens, such as the synthetic peptide of malaria, can be attached to larger proteins or other carriers to increase the immune response. The relationship between molecular size and complexity of an antigen relative to immunogenicity reflects the availability of antigenic determinants on the molecule. This relationship was first noted by Landsteiner when he demonstrated the need to complex small radicals with larger (carrier) molecules to stimulate an immune response. However, the mechanistic basis for the requirement was to await experiments that demonstrated the carrier effect and the need for a minimum of two antigenic determinants on a molecule to express immunogenicity. These determinants represented the carrier and haptenic determinants that interact with T and B lymphocytes, respectively. However, the influence of the carrier moiety extends beyond simple antigenicity through activation of T cells in T-dependent humoral responses.

The combination of determinants on an antigen molecule can influence the immune response by differential activation of various types of helper and suppressor T cells. A model system demonstrating this effect is the genetically controlled humoral response of responder (C57B1/6) and non-responder (DBA/l ) mice to the synthetic terpolymer l-glutamic acid⁶⁰-L-alanine³⁰-L-tyrosine¹⁰ (GAT). While C57B1/6 mice respond to this polypeptide, DBA/l mice will respond only if the GAT is coupled to methylated bovine serum albumin (MBSA). However, if the mice are injected with GAT prior to immunization with GAT-MSBA, a detectable antibody response to GAT does not occur. The explanation for these observations is that GAT stimulates helper T cells in the responder mice but preferentially activates suppressor T cells in non-responder mice. This predominance of suppressor cells prevents a response to GAT even when coupled to MBSA. However, if primary immunization is with GAT-MBSA, activation of helper T cells by the carrier moiety provides help that overrides the effect of any suppressor cells activated by GAT.

Determinants associated with a native protein molecule have also been demonstrated to contribute differently to help and suppression. Conjugation of an immunogenic carrier to an antigen can change the isotype of antibodies produced in response to that antigen. Purified polysaccharides from many encapsulated bacteria are thymus-independent antigens due to their polymeric nature with multiple repeating antigenic determinants. While they represent protective antigens of these bacteria, the IgM antibodies produced have limited efficacy in preventing disease. This is largely due to their inability to stimulate immunologic memory or adequate immune responses in very young or old individuals who are at high risk from the infections. Therefore, polysaccharides from *Neisseria meningitidis* and *Haemophilus influenza* type b have been conjugated to proteins, such as tetanus toxoid. These conjugated preparations act as thymus-dependent antigens and induce IgG responses to the polysaccharide moiety as well as immunologic memory. They also induce responses in young or old individuals. Likewise, the thymic-independent polysaccharide carriers have little potential for enhancing the immunogenicity of peptides, such as those involved with malaria which require thymic-dependent IgG immune responses.

Publications by Feldmann and Lee and others state that flagella antigens of *Salmonella* organisms are typical thymic-independent antigens which stimulate strong IgM antibody responses.^{2,3} They stimulate only late maturing B cells which are absent from infants. Such immunogens also tend to induce tolerance in infants and do not induce memory or other aspects of the complex immune responses induced by thymic-dependent antigens in adults. This published data would lead one to believe that they have little potential as adjuvants or carriers for malaria peptides or other small antigens which require thymic-dependent IgG antibody responses.

There probably is no precise point of transition that distinguishes a carrier from an adjuvant. The carrier moiety is contributory to a property of antigens that has been termed intrinsic adjuvanticity. The capacity of certain materials to convert a tolerogen to an immunogen has been termed as extrinsic adjuvanticity. Adjuvanticity can be enhanced by increasing the size of the antigen through aggregation of proteins or adsorption to immunogenic or inert carriers. Thus materials, such as aluminum hydroxide, latex particles, bentonite, or liposomes that adsorb antigen and enhance the immune response, are termed adjuvants. However, this observed effect of aggregation of antigen represents only a limited view of adjuvant actions which are now recognized as being extremely complex.

Small peptides and other haptens are incapable of evoking a strong immune response without the use of an adjuvant. Most adjuvants that are currently available are toxic and/or do not evoke an immune response that is effective in protecting the animal or human against infection with the infectious agent. Thus, what is needed is a vaccine which can be administered to an animal or human and will cause the immune system to mount a prolonged and potent immune response of the correct type against an appropriate antigen.

Large hydrophobic nonionic block copolymer surfactants have been shown to be effective immunologic adjuvants which are potentially useful in man.^{4,5,6} They appear to act as adhesives which bind protein antigens to the surface of oil drops and/or cells in a way which facilitates antigen presentation. Previous studies have demonstrated that these copolymers can induce high titer, long lasting antibody responses. Interestingly, closely related copolymers have only weak activity, are not adjuvants, or induce inappropriate responses or tolerance. This makes prediction of adjuvant activity complex and imprecise.

One might predict that adjuvants whose primary activity was cell stimulation or immunomodulation might work well in combination with the adhesive copolymer adjuvants. The combination of copolymer P 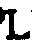 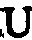 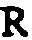 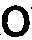 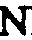 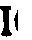 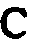 ® L121 with a threonyl derivative of MDP has been reported to induce better response, particularly a cell mediated immune response, than L121 by itself.⁷

Lipopolysaccharides are well-known as B cell mitogens with pronounced effects on macrophages.⁸ Its adjuvant activities have been know for many years, but its use has been limited by toxicity and variable efficacy. It has been reported in several articles and reviews that the biological activity of the lipopolysaccharides resides in the lipid A portion of the lipopolysaccharide molecule.⁹

The isotype of antibody is very important in resistance to many infections, but little is known about how to produce a particular isotype response. IgG2a has been associated with being a protective isotype for a variety of pathogens, including trypanosoma cruzi,^{10,11} *T. musculi*¹² and *plazmodium Yoelii* (malaria) and the bacterium Brucella. IgE antibodies are particularly toxic for parasites in mice. Many parasites including helminths, schistosomes, and nematode larvae naturally stimulate predominantly IgG1 and IgE antibodies. The production of IgG1 and IgE appear to be linked. Each isotype has fictional advantages which may be appropriate for neutralizing a particular infectious agent. IgG2a binds most avidly to macrophages, which may influence antibody dependent cell mediated cytotoxicity and phagocytosis and can activate complement. The murine IgG3 isotype is particularly effective in protecting against infections with encapsulated bacteria such as *S. pneumoniae.*

Finally, diseases caused by *Streptococcus pneumoniae* are among the most important bacterial infections of infancy and childhood. A multivalent vaccine containing capsular polysaccharides from 23 types of pneumococci is widely used today. Several studies show that the efficacy of the vaccine in preventing bacteremic illness was 0% in children 2-10 years of age and 49% in persons older than 10 years. There is no convincing evidence that the vaccine is effective for the chronically ill and studies have shown that there is no benefit for the elderly and the institutionalized patients.

By themselves, capsular polysaccharides are thymus independent type 2 (TI-2) antigens which are poorly immunogenic in the very young or very old. TI-2 antigens induce only a restricted number of isotypes, mainly IgM. They induce only a weak memory response, or no memory response, and tolerance is easily induced.

In Abstracts of the 90th Annual Meeting of the American Society for Microbiology, 13-17 May 1990, page 130, abstract No. E-65, the use of block copolymers designated as Pluronic® L121, L141 and L180,5 as adjuvants in vaccine compositions is disclosed.

From US-A-4,770,874 an adjuvant composition containing an immunopotentiating amount of an immunostimulating glycopeptide, a polyoxypropylene-polyoxyethylene block copolymer, for example Pluronic® L101, L121 and L122, a glycol ether-based surfactant and optionally a metabolizable non-toxic oil is known.

In US-A-4,609,546 a composition wherein a physiologically active polypeptide or a glycoprotein of human origin, for instance, urokinase, kallikrein or leukocyte interferon is coupled to a polyoxyethylene-polyoxypropylene copolymer is disclosed.

US-A-4,606,918 refers to an adjuvant vehicle for vaccines comprising an immunopotentiating amount of glycopeptide, a multi-phase forming amount of an non-toxic polyoxypropylene-polyoxyethylene block copolymer, a multi-phase stabilizing amount of a glycol ether-based surfactant, and optionally a metabolizable oil of 6-30 carbon atoms, and buffered isoosmotic aqueous solution in a quantity sufficient to make volume.

In Infection and Immunity 56, 5, 1988, page 1391-1393, it is disclosed that nonionic block polymer surfactants like copolymers of polyoxypropylene and polyoxyethylene, enhance the immunogenicity of pneumococcal hexasaccharide-protein vaccines.

The object of the present invention in view of the prior art is to provide a vaccine comprising an adjuvant capable of inducing a longer lasting high titer of antibodies.

This object has been attained by a surface-active copolymer with the following formula:

HO(C₂H₄O)_{b}(C₃H₆O)ₐ(C₂H₄O)_{b}H,

wherein the molecular weight of the hydrophobe (C₃H₆O) is between approximately 5200 to 9000 and the percentage of hydrophile (C₂H₄O) is between approximately 3% and 15% by weight, whereby a copolymer in accordance with the above formula having a molecular weight of the hydrophobe of approximately 5200 and having the hydrophile percentage of the total molecular weight of 5% is excluded for use in a vaccine, and by a vaccine composition comprising the above identified surface-active copolymer.

The present invention comprises a vaccine adjuvant which, when admixed with an antigen and administered into a human or animal, will induce a more intense immune response to the antigen than when the antigen is administered alone. In many cases, the adjuvant that is described as the present invention will increase overall titer of antibodies specific for the vaccine antigen. For example, when the present invention is practiced with a conventional antigen, the isotype that is induced is changed from a predominantly IgG1 isotype to the more protective IgG2 isotype and, in some cases, IgG3 isotype or the corresponding isotype in other species. Thus, by practicing the present invention, one can improve the overall protective effect of conventional vaccines.

In addition, the present invention is particularly effective in inducing protective antibodies against peptide antigens including, but not limited to, (asparagine-alanine-glycine-glycine)5-tyrosine [(NAGG)₅] malaria antigen. It is effective for a wide range of antigens and types of antigens. This includes polysaccharides, such as pneumococcal polysaccharide, oligosaccharides, proteins, peptides, and natural or synthetic haptens or combinations of these materials.

Another object of the present invention is to provide a vaccine which induces stronger antibody responses to antigens in infants and young children and in aged people who respond poorly to conventional vaccines.

Another object of the present invention is to provide an adjuvant that will induce desired isotypes of antibodies.

Another object of the present invention is to provide an adjuvant and vaccine which will induce protective immune responses in very young and aged individuals who respond poorly to conventional vaccines.

Another object of the present invention is to provide an adjuvant and vaccine which will induce an appropriate balance of antibody and cell mediated immunity thereby providing the maximum protection against a particular disease.

Another object of the present invention is to provide an adjuvant that will induce longer lasting antibody populations.

Another object of the present invention is to provide a effective vaccine that can utilize a recombinant protein or a synthetic peptide to produce a sustained immune response capable of protecting an individual from infection by the malaria parasite.

Another object of the present invention is to provide an effective vaccine that can utilize a synthetic peptide of the AIDS virus to produce an immune response that is effective in preventing the disease.

Yet another object of the present invention is to provide a vaccine that is capable of stimulating the immune system of an animal or human to produce a potent and prolonged IgG response to a small immunogenic determinant, such as a peptide, hapten or polysaccharide or a large molecule such as a protein or polysaccharide.

Another object of the present invention is to provide a vaccine which has very low toxicity for humans or animals.

Yet another object of the present invention is to provide a vaccine which causes little or no local allergic reaction.

A further object of the present invention is to provide a vaccine which can be lyophilized.

Another object of the present invention is to provide a replacement for Freund's Complete Adjuvant for the production of antibodies in animals.

It is yet another object of the present invention to provide an adjuvant that will induce desired antibody isotypes.

Another object of the present invention is to provide an adjuvant that can be used with a conventional vaccine preparation.

These and other objects, features and advantages of the present invention will become apparent after a review of the following detailed description of the disclosed embodiment and the appended claims.

### Brief Description of the Drawing

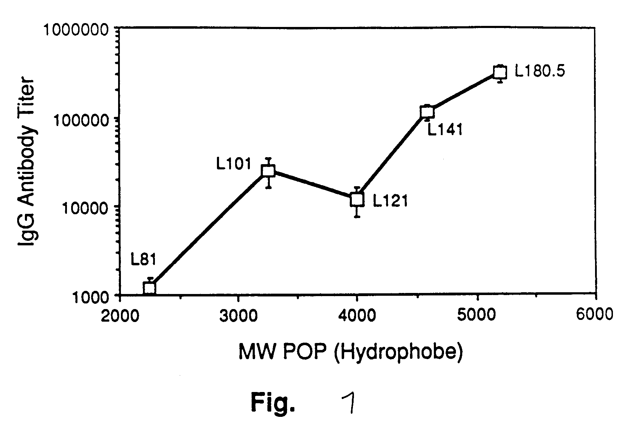

Fig. 1 is a graph comparing copolymer adjuvants administered with soluble antigen, TNP₁₀HEA.

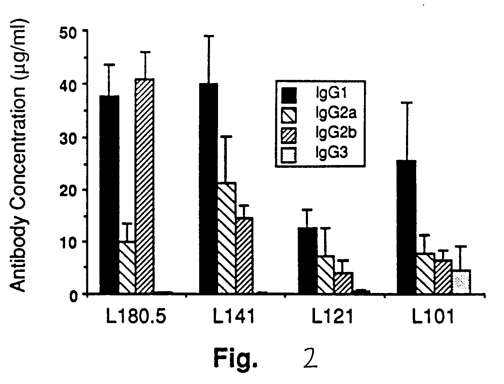

Fig. 2 shows the influence of molecular weight of POP on antibody titer to TNP₁₀HEA.

### Detailed Description

The present invention comprises an improved adjuvant. In one embodiment of the present invention, an antigen is admixed with an effective amount of an adjuvant, the adjuvant comprises a surface-active copolymer having the following general formula:

HO(C₂H₄O) _{b}(C₃H₆O)ₐ (C₂H₄O)_{b}H

wherein the molecular weight of the hydrophobe (C₃H₆O) is between approximately 5200 to 9000 and the percentage of hydrophile (C₂H₄O) is between approximately 3% and 15% by weight whereby a copolymer in accordance with the above formula having a molecular weight of the hydrophobe of approximately 5200 and having the hydrophile percentage of the total molecular weight of 5% is excluded. The copolymers may be obtained from BASF Corporation, Parsippany, New Jersey or from CytRx Corporation, Atlanta, GA.

Another preferred surface-active copolymer is a copolymer designated P 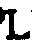 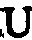 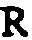 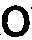 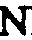 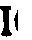 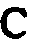 ® L181.5 with the following formula:

HO(C₂H₄O)_{b}(C₃H₆O)ₐ(C₂H₄O)_{b}H

wherein the molecular weight of the hydrophobe (C₃H₆O) is approximately 5200 and the percentage of hydrophile (C₂H₄O) is approximately 15% by weight.

Another preferred surface-active copolymer is a copolymer designated P 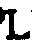 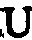 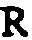 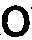 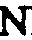 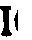 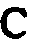 ® L190.5 with the following formula:

HO(C₂H₄O)_{b}(C₃H₆O)ₐ(C₂H₄O)_{b}H

wherein the molecular weight of the hydrophobe (C₃H₆O) is approximately 8600 and the percentage of hydrophile (C₂H₄O) is approximately 5% by weight.

An adjuvant formulation which is contemplated as part of the present invention is comprised of oil such as animal oil, such as squalane or squalene, vegetable oil or mineral oil, a non-ionic surface active agent suitable for forming water-in-oil emulsions such as Span 80 (sorbitan monooleate), silica and a surface active copolymer with the following general formula:

HO(C₂H₄O)_{b}(C₃H₆O)ₐ(C₂H₄O)_{b}H

as defined above

The preferred amounts of the components are approximately 40% to 90% by weight of squalene, 2% to 50% by weight sorbitan monooleate, approximately 0.5 to 10% by weight of silica and approximately 2% to 10% by weight of the surface active copolymer. The silica particles are preferably approximately 0.5 to 20 µ in diameter.

The adjuvant comprising a combination of LPS and surface-active copolymer produces a synergy of effects both in terms of peak titer and time to reach peak titer. In some cases, especially with the lower molecular weight lipopolysaccharides, the initial titer is higher and then is slightly depressed with time. With the higher molecular weight lipopolysaccharides, the initial titer is higher and the response remains high over time. With all of the lipopolysaccharides, the combination tends to increase the protective IgG2a and IgG2b isotypes. This is unexpected because the LPS, by itself, has been reported to act as an adjuvant to induce a predominantly IgG1 immune response.

The improved adjuvant also comprises a surface-active copolymer with the following general formula:

HO(C₂H₄O)_{b}(C₃H₆O)ₐ(C₂H₄O)_{b}H

according to the invention and a reverse octablock copolymer with the following general formula: 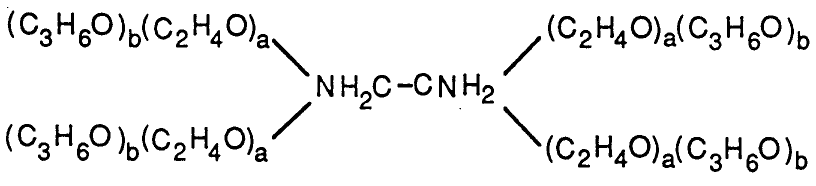 wherein:
the molecular weight of the hydrophobe portion of the octablock copolymer consisting of (C₃H₆O) is between approximately 5000 and 7000 daltons;
a is a number such that the hydrophile portion represented by (C₂H₄O) constitutes between approximately 10% and 40% of the total molecular weight of the compound,
b is a number such that the (C₃H₆O) portion of the octablock copolymer constitutes between approximately 60% and 90% of the compound.

The (C₃H₆O) portion of the copolymer can constitute up to 95% of the compound. The (C₂H₄O) portion of the copolymer can constitute as low as 5% of the compound.
a is a number such that the hydrophile portion represented by polyoxyethylene (C₂H₄O) constitutes between approximately 5% to 40% of the total molecular weight of the compound,
the mean aggregate molecular weight of the hydrophobe portion of the octablock copolymer consisting of polyoxypropylene (C₃H₆O) is between approximately 4000 and 8000 daltons; and b is a number such that the polyoxypropylene (C₃H₆O) portion of the total molecular weight of the octablock copolymer constitutes between approximately 60% and 90%.

The (C₃H₆O) portion of the copolymer can constitute up to 95% of the compound The (C₂H₄O) portion of the copolymer can constitute as low as 5% of the compound.

The present invention also includes vaccines which comprise antigens and the aforementioned adjuvants.

The present invention also comprises a vaccine that is especially useful for immunizing an animal or human against a polysaccharide, protein, small peptide or other hapten. According to the present invention, the small peptide or hapten is conjugated to flagella that is derived from a microorganism. The flagella may be derived from any flagellated microorganism; however, those from *Salmonella species* are preferred. It is to be understood that the preferred bacterial species from which the flagella are derived for any particular application is dependent upon the particular antigen requirements of the application and is not critical for this invention.

Some bacteria possess a single flagellum while others have a tuft of flagella and still others have flagella distributed over the entire cell surface. Bacterial flagella are between 10 and 35 nm in diameter and may sometimes exceed 10 to 15 µm in length, or many times the diameter of the cell. Most bacterial flagella show a regular and uniform curl with a wavelength of about 2.5 µm.

When bacterial flagella, which are protein in nature, are acidified to pH=3, they dissociate into identical monomeric subunits called flagellin, which has a molecular weight of approximately 40,000 in most species. Under appropriate conditions of pH and salt concentration, flagellin monomers will spontaneously reaggregate to form structures that appear to be identical with intact flagella possessing periodic curls of the same wavelength as the native flagella.

Intact bacterial flagella in the native form or fixed with a number of fixative agents can be used in practicing the present invention. Additionally, repolymerized flagellin is satisfactory in practicing the present invention. It is believed that an essential component of the present invention is that the preparation consists of a polymer composed of flagellin molecules regularly spaced in a geometric pattern to produce the elongated flagellar structure typical of the particular microorganism.

A number of procedures for preparing flagella from bacterial cultures have been developed and are well-known to those of ordinary skill in the art. The preferred procedure is a modification of the procedure of Kobayashi, *et al*., as described herein.¹³

*Salmonella typhi* organisms of strain TY2 are grown in motility agar. The highly motile organisms should be selected because they produced the most flagella. Organisms are then inoculated in 20 titers of trypticase soy broth and incubated at 37°C for approximately 30 hours until the end of the log phase of growth. The organisms may be killed at this time by the addition of formaldehyde to produce a 0.3% suspension. The organisms are preferably collected by centrifugation; however, care should be taken to avoid production of excessive shear force. The flagella are then removed from the organisms by shaking vigorously for 20 minutes in a shaker. Other mixes and devices which produce a shear force to break off the flagella without disrupting the organism are equally satisfactory.

The flagella are then separated from the cell bodies by differential centrifugation. The cell bodies are removed by centrifuging at approximately 2000 rpm in a standard laboratory centrifuge. The flagella are then collected by ultracentrifugation at 30,000 rpm. The flagella are then resuspended and recentrifuged in an ultracentrifuge, and soluble contaminating materials are poured off. Large contaminating materials will form a black spot at the bottom of the transparent flagella pellet. This material is physically removed and discarded. The end product derived from 20 liters of bacterial culture will be approximately 100 mg of purified flagella.

Flagellin may be produced by acidifying unfixed flagella at a pH of approximately 2 overnight. This treatment dissociates the flagellar proteins to produce the monomers of flagellin which have a molecular weight of approximately 30,000. The monomers reassemble into the polymerized flagella when allowed to stand at neutral pH for a period of at least 24 hours. The repolymerized flagellin is nearly as effective as the native flagella as an adjuvant and carrier for small antigen moieties. The monomeric flagellin or proteolytic cleavage fragments of flagellin protein are very much less effective.

The antigen, *i.e*., protein, polysaccharide, hapten or peptide moieties, can be chemically conjugated to the flagella by any one of the standard means well known to those of ordinary skill in the art. One of the simplest and most effective means is by using gluteraldehyde. Gluteraldehyde is a divalent cross-linking compound which covalently attaches the peptide to the flagella and further fixes the flagella preparation. Other chemical cross-linking reagents or chemical antigen derivatives, such as dinitrofluorobenzene, are effective. The methods of conjugating an antigen, hapten or peptide moieties are well known to those of ordinary skill in the art.

The amounts of antigen attached to the flagella varies with the particular application and is not a critical component of this invention. Preferably, between 2 and 10 peptide or hapten units per flagellin monomer in the flagella preparation is sufficient. Smaller multiples are needed for larger protein or polysaccharide antigens.

The conjugated flagella preparation is purified by dialysis, centrifugation, or any other standard method. The material is then resuspended in saline at a concentration approximating 100 µg/ml.

This preparation is effective in low doses between 1 and 100 µg per injection. A dose of 10 µg produces a satisfactory response in many situations. The material can be injected by any convenient route, intravenous, subcutaneous, intramuscular, or intraperitoneal. The subcutaneous or intramuscular mute is usually the most convenient for many vaccine purposes.

As an example, injections of 20 µg of *Salmonella typhi* flagella conjugated with dinitrophenol resulted in IgG antibody titers specific for the hapten DNP which rose at the end of the first week after injection and persisted for over one year.

Persistence of the immune response to flagella and to antigenic moieties conjugated to flagella is unusual and unexpected. The material does not form a local depot of antigen at the site of injection. Approximately 90 to 95% of the injected dose of flagella is broken down and excreted within 24 hours. A portion of the material is retained for a prolonged time in germinal centers within local lymph nodes. It is believed that the presence of this antigen in germinal centers is responsible for the prolonged antibody production.

This invention has numerous advantages over other available adjuvant preparations. It produces very little inflammation at the site of injection and is entirely biodegradable. This contrasts sharply with oil emulsions or mineral salts, such as aluminum. Very small doses of antigen are required to produce prolonged immune responses. A significant portion of the antibody is complement-fixing IgG which is the type required for protection against malaria, sporozoites, and other important infections. The product is stable especially when prepared with fixatives, such as gluteraldehyde. It can be lyophilized and stored at room temperature indefinitely. When reconstituted with saline, it is stable for several weeks with refrigeration and several days at room temperature.

Unlike live attenuated vaccines which may produce infections in susceptible hosts, this vaccine preparation consists only of polymerized protein with traces of polysaccharide.

The preferred dose of a vaccine prepared according to the present invention is between 5 µg and 500 µg. The optimal dose for any vaccine will depend upon the antigen that is conjugated with the flagella protein and the immunological condition of the animal or human that is being vaccinated.

A preferred adjuvant has the following formula:

HO(C₂H₄O)_{b}(C₃H₆O)ₐ(C₂H₄O)_{b}H

wherein the molecular weight of the hydrophobe (C₃H₆O) is approximately 5200 and the percentage of hydrophile (C₂H₄O) is approximately 10% by weight.

The polymer blocks are formed by condensation of ethylene oxide and propylene oxide onto a tetrafunctional ethylene diamine initiator at elevated temperature and pressure in the presence of a basic catalyst. There is some statistical variation in the number of monomer units which combine to form a polymer chain in each copolymer. The molecular weights given are approximations of the average weight of copolymer molecule in each preparation. A further description of the preparation of these block copolymers is found in U.S. Patent No. 2,674,619 and U.S. Patent No. 2,979,528 ¹⁴

The published molecular weight for poloxamers and poloxamines is commonly determined by the hydroxyl method. The end groups of polyether chains are hydroxyl groups. The number averaged molecular weight can be calculated from the analytically determined "OH Number" expressed in mg KOH/g sample. It should be understood that the absolute value of the molecular weight of a polydisperse compound can be different depending upon the methodology used to determine the molecular weight. Thus, it is important to know by what method the molecular weight of the copolymer has been determined. As used herein, the molecular weights of all of the copolymers was determined by the hydroxyl method. A slightly different number is obtained when the molecular weight is determined by another method such as high performance liquid chromatography.

The vaccine which comprises the present invention is mixed with the octablock copolymer and administered to the human or animal. The preferred amount of adjuvant administered with the vaccine of the present invention is between approximately 0.1 mg and 5.0 mg with the most preferred amount between approximately 0.5 mg and 2 mg.

Another embodiment of the adjuvants of the present invention are various derivatives of lipid A. The structures of the various species of lipid A are described in articles by Takayama, K., *et al*. and Raetz, C.R.H., both of which are incorporated herein by reference.^{15,16} Monophosphoryl lipid A has lower toxicity than the complete lipid A molecule but has a lower toxicity to animals than does the complete lipid A. Lipid IVA and lipid X are precursors in the biosynthesis of lipid A. The structures of some of the lipid A derivatives that are contemplated as part of the present invention are shown in Figs. 10 through 13.

Several recent reports have implicated IgG2a antibodies as conferring protection against several veal and bacterial infections. IgG2b antibody has been less well studied but has also been reported to be protective. Antibody of the IgG1 subclass does not fix complement and is thought to be of considerably less protective efficacy in many situations. Consequently, the ability of LPS derivatives to shift the antibody response toward the IgG2 isotypes, especially when admixed with copolymers, can be expected to increase the efficacy of vaccines.

Antigens that can be used in the present invention are compounds which, when introduced into a mammal, will result in the formation of antibodies. Representative of the antigens that can be used according to the present invention include, but are not limited to, natural, recombinant or synthetic products derived from viruses, bacteria, fungi, parasites and other infectious agents in addition to autoimmune diseases, hormones or tumor antigens which might be used in prophylactic or therapeutic vaccines. The viral or bacterial products can be components which the organism produced by enzymatic cleavage or can be components of the organism that were produced by recombinant DNA techniques that are well-known to those of ordinary skill in the art. The following is a partial list of representative antigens:

### Viruses

HIV
Rotavirus
Foot and mouth disease
Influenza
Parainfluenza
Herpes species, Herpes simplex, Epstein Barr virus Chicken pox, pseudorabies
Rabies
Polio
Hepatitis A
Hepatitis B
Hepatitis C
Measles
Distemper
Venezuelan equine encephalomyelitis
Rota virus
Feline leukemia virus
Reovirus
Respiratory sycytial virus
Lassa fever virus
Polyoma tumor virus
Canine parvovirus
Bovine papilloma virus
Tick borne encephalitis
Rinderpest
Human rhinovirus species
Enterovirus species, Mengo virus
Paramyxovirus
Avian infectious bronchitis virus

### Bacteria

*Bordetella pertussis*
*Brucella abortis*
*Escherichia coli*
*Salmonella species, salmonella typhi*
Streptococci
Cholera
Shigella
Pseudomonas
Tuberculosis
Leprosy

### Ricketsial Infections

Rocky mountain spotted fever
Thyphus

### Parasites

Malaria (*Plasmodium. falciparum, P. vivax, P. malariae*)
Schistosomes
Trypanosomes

### Fungus

*Cryptococcus neoformans*

### Subunit recombinant proteins

Herpes simplex
Epstein Barr virus
Hepatitis B
Pseudorabies
Flavivirus, Denge, Yellow fever
*Neisseria gonorrhoeae*
Malaria: circumsporozoite protein, merozoite protein
Trypanosome surface antigen protein
Pertussis
Alphaviruses
Adenovirus

### Proteins

Diphtheria toxoid
Tetanus toxoid
Meningococcal outer membrane protein (OMP)
Streptococcal M protein
Hepatitis B
Influenza hemagglutinin

### Synthetic peptide

Malaria
Influenza
Foot and mouth disease virus
Hepatitis B, Hepatitis C

### Polysaccharide

Pneumococcal polysaccharide
*Haemophilis influenza* polyribosyl-ribitolphosphate (PRP)
*Neisseria meningitides*
*Pseudomonas aeruginosa*
*Klebsiella pneumoniae*

### Oligosaccharide

Pneumococcal

Haptens are compounds which, when bound to an immunogenic carrier and introduced into a chordate, will elicit formation of antibodies specific for the hapten. Representative of the haptens are steroids such as estrogens and cortisones, low molecular weight peptides, other low molecular weight biological compounds, drugs such as antibiotics and chemotherapeutic compounds, industrial pollutants, flavoring agents, food additives, and food contaminants, and/or their metabolites or derivatives.

In addition to the foregoing embodiments of the present invention, addition of certain of the copolymers to silica suspensions has provided an unexpected ink in the adjuvant activity of the composition.

Silica is a known adjuvant, but its use has been limited by toxicity, especially fibrosis. This toxicity is reduced and the effectiveness increased by incorporation of the silica into an oily vehicle with or without other adjuvant moieties such as surface-active copolymers or LPS. The dose and toxicity of silica are reduced, while the effectiveness is increased by the present invention. Preferably, the oil emulsion comprises an oil and silica particles with the emulsion comprising between 40% and 99% oil. A preferred oil is squalane (Sigma Chemical Company, St. Louis, MO). In addition to the oil, one can optionally add a detergent or mixture of detergents to the oil. Examples of detergents that can be used in the present invention are polyoxyethylenesorbitan (Tween) and sorbitan (Span) (Sigma Chemical Company, St. Louis, MO).

Since, certain components of vaccine adjuvants are liable to oxidation, antioxidants have been included as preservatives. The oil vehicle squalene is particularly susceptible to oxidation. The block copolymers may also be affected. Many antioxidants are available which are potentially acceptable to prevent oxidative degradation of vaccine components. Examples of these, tocopherol (vitamin E) or tocopherol derivatives, were found to have the ability to enhance adjuvant activity in addition to preventing oxidation. It has been found that the antioxidants are particularly effective in increasing immune responses and reducing local inflammation in addition to serving as an antioxidant when used in combination with the block copolymer or silica emulsions. Thus, it is contemplated as part of the present invention the admixture of antioxidants, such as tocopherol or tocopherol derivatives, with the adjuvants and vaccines described herein.

The following specific examples will illustrate the invention as it applies to enhancing the immune response of an organism to small haptens. It will be appreciated that other examples will be apparent to those of ordinary skill in the art and that the the invention is not limited to these specific illustrative examples.

### Example

Groups of five to ten mice were immunized with TNP-HEA in a 2% squalane-water-water emulsion containing 1 mg of each of the copolymers indicated in Fig. 7. The time courses of the antibody responses are similar in each of the groups except L81* which induces only a transient response. The titers peak at approximately one month after injection and persisted for over three months. The animals are boosted on day 90 after immunization. They are bled again one week post boost. The copolymers with 10% or less polyoxyethylene (POE) all induced strong immune responses. Copolymer L122* is a poor adjuvant. The adjuvant activity of copolymers with a range of POE chain lengths and the polyoxypropylene (POP) chains with molecular weights of 5200 (L180.5*, L181.5 and L182.5) follow the pattern established previously for the series of smaller copolymers L121*, L122* and L123*. Copolymers with more than 10% POE are again found to be ineffective adjuvants.
* not a blockcopolymer of the present invention

The mean titers stimulated by the copolymers with 10% or less POE of each length of POP chain increases with increasing molecular weight of the POP hydrophobe as shown in Fig. 7. While there is variability between and among groups, the general pattern of increasing titer with increasing molecular weight of hydrophobe is observed repeatedly.

The isotype of antibody is measured at multiple time points using a ELISA assay with calibrated class specific atisera. As shown in Fig. 8, the copolymer preparations which were effective adjuvants for inducing antibody induced distinctly different patterns of isotype. The lower molecular weight preparation, L101*, induces a predominant IgG1 response with lesser amounts of IgG2a and IgG2b. Increasing molecular weight of the hydrophobe increases the proportion of IgG2, especially IgG2b. Interestingly, the production of the IgG3 isotype follows the opposite pattern with the highest titers produced by the lower molecular weight preparations, L121* and especially L101*. The ratio of IgG1 to IgG2b antibody increases in a nearly linear fashion with molecular weight of the hydrophobe as shown in Fig. 8. The distribution of isotypes is measured at multiple intervals following the 28 day determination. The isotype patterns produced by each copolymer tend to persist during subsequent assays.
* not a blockcopolymer of the present invention

### Example 29

¹Clark, W.R., The Experimental Foundations of Modern Immunology, Chapter 4, "The properties and fine structure of immunoglobulins", pgs. 62-74. John Wiley & Sons
²Feldmann, M, *et al.,* "The Relationship Between Antigenic Structure and the Requirement for Thymus-Derived Cells in the Immune Response". *J. Exp. Med.,* Vol. 134, pgs. 103-119 (1971)
³Lee, *et al.,* "Decline and Spontaneous Recovery of the Monoclonal Response to Phosphorylcholine During Repeated Immunization", *J. Immun.,* Vol. 113, pgs. 1644-1646 (1974)
⁴ Hunter, R.L., *et al.,* "The Adjuvant Activity of Nonionic Block Polymer Surfactants I. The Role of Hydrophile-Lipophile Balance", *J. Immunol.;* Vol. 127, pgs. 1244-1250 (1981)
⁵Hunter, R.L., *et al.,* "The Adjuvant Activity of Nonionic Block Polymer Surfactants II. Antibody Formulation and Inflammation Related to the Structure of Triblock and Octablock Copolymers", *J. Immunol.,* Vol. 133, pgs. 3167-3175 (1984)
⁶Hunter, R.L., *et al.,* "The Adjuvant Activity of Nonionic Block Polymer Surfactants III. Characterization of Selected Biologically Active Surfaces", *Scand. J. Immunol.,* Vol. 23, pgs. 287-300 (1986)
⁷See U.S. Patent Numbers 4,606,918 and 4,770,874
⁸Louis, J.A., *et al.,* Lipopolysaccharides: From Immunostimulation to Autoimmunity, *Springer Seminars in Immunopathology,* Vol. 2, pgs. 215-229 (1979)
⁹For example, Galanos, C., *et al.,* "Biological Activities and Immunological Properties of Lipid A", *Microbiology,* pgs. 269-276 (1977)
¹⁰ Scott, M.T., *et al.,* "Restricted IgG isotype profiles in T. cruzi infected mice and Chagas' disease patients". *Clin. Exp. Immunol.,* Vol. 58, pgs. 372-379 (1984)
¹¹Takehara, *et al.,* "Trypanosoma cruzi: role of different antibody classes in protection against infection in the mouse", *Exp. Parasitology,* Vol. 52, pgs. 137-146 (1981)
¹²Wechsler,*et al.,* "Heat labile IgG2a antibodies affect cure of Trypanosoma muculi infection in C57BL/6 mice", *J. Immunol.* Vol. 137, pgs. 2968-2972 (1986)
¹³Kobayashi, *et al., Arch. Biochem. Biophys.* 84, pgs. 342-362 (1959)
¹⁴ Also see Schmolka, I.R., "A Review of Block Polymer Surfactants, *J. Am. Oil Chemists' Soc*., 54:110-116 (1977) and Block and Graft Copolymerization, Vol. 2, edited by R.J. Ceresa, John Wiley & Sons, New York (1976)
¹⁵Takayama, K., *et al.,* "Fatty Acyl Derivatives of Glucosamine 1 Phosphate in *Escherichia coli* and Their Relation to Lipid A", *J. Biol. Chem.,*Vol. 256, pgs. 7379-7385 (1983)
¹⁶Raetz, C.R.H., "Structure and Biosynthesis of Lipid A in *Escherichia coli,* in *Escherichia Coli* and *Salmonella Typhimurium*", *Cellular and Molecular Biology,* Vol. 1, Neidhardt, F.C. Editor, American Society for Microbiology
¹⁷Qureshi, N., *et al.,* "Purification and structural determination of nontoxic lipid A obtained from the lipopolysaccharide of *Salmonella typhimurium*", *J. Biol. Chem.,* Vol. 257, pgs. 11808-11805 (1982)
¹⁸Louis, *et al., supra*
¹⁹Saunders, G.C., "The art of solid phase enzyme immunoassay including selected protocols", *Immunoassays in the Clinical Laboratory,* Alan R. Liss, New York, pgs. 111-112 (1979)
²⁰Rietchsel, E., *et al.,* "Bacterial Endotoxins: Relationships Between Chemical Structure and Biological Activity", *Immunologic Adjuvants and Vaccines,* Vol. 179, Ed. by Gregoriadis, G., *et al.,* Plenum Press, pgs. 61-74
²¹Justine S.G., *et al., Methods in Immunology,* 3rd Ed., Chapter 18, pgs. 153-158 (1977)
²²Takayama, K., *et al.,* "Fatty acyl derivatives of glucosamine 1-phosphate in *Escherichia coli* and their relation to lipid A. Complete structure of a diacyl GlcN-1-P found in a phosphatidylglycerol-deficient mutant", *J. Biol. Chem.,* Vol. 258, pgs. 7379-7385 (1983)
²³Raetz, C.R.H., *et al.,* "Isolation and characterization of eight lipid A precursors from a 3-deoxy-D-manno-octylosonic acid deficient mutant of Salmonella typhimurium*", J. Biol Chem.,* Vol. 260, pgs. 16080-16088 (1985)
²⁴Qureshi, *et al.,* "Complete structural determination of lipolysaccharide obtained from deep rough mutant of Escherichia coli. Purification by high performance liquid chromatography and direct analysis by plasma desorption mass spectrometry." *J. Biol. Chem.,* Vol 263, pgs. 11971-11976 (1988)
²⁵Qureshi, N., *et al.,* "Purification and structural determination of nontoxic lipid A obtained from the lipopolysaccharide of Salmonella typhimurium", *J. Biol. Chem.,* Vol. 257, pgs. 11808-11815 (1982)
²⁶Galanos, C., *et al.,* "A new method for the extraction of lipopolysaccharides", *Eur. J. Biochem.,* Vol. 9, pgs. 245-249 (1969)
²⁷Qureshi, N., *et al.,* "Position of ester groups in the lipid A backbone of lipopolysaccharides obtained from *Salmonella typhimurium", J. Biol. Chem.,* Vol. 258. pgs. 12947-12951 (1983)
²⁸Westphal, *et al.,* "Extraction with phenol-water and further applications of the procedure", *Methods Carbohydrate Chem.* Vol. 5, pgs. 83-91 (1965)
²⁹Vukajlovich, *et al.,* "Conversion of lipopolysaccharides to molecular aggregates with reduced subunit heterogeneity: Demonstration of LPS-responsiveness in 'Endotoxin-unresponsive C3H/HeJ splenocytes'", *J. Immunol.,* Vol. 130, pgs. 2804-2808 (1983)
³⁰Saunders, *supra*
³¹Lowry, O.H., *et al.,* "Protein measurement with the folin phenol reagent", *J. Biol. Chem..,* Vol. 193, pgs. 265-275 (1951)
³²Liang *et al.,* "Oral Administration of Cholera Toxin-Sedai Virus Conjugate Potentiates Gut and Respiratory Immunity Against Sendai Virus", *J. Immunol.,* Vol. 141, No. 5, pgs. 1495-1501 (1988)
³³Justine S.G., *et al., supra*

## Claims

1. A surface-active copolymer for use in a vaccine with the following formula:
HO(C₂H₄O)_{b}(C₃H₆O)ₐ(C₂H₄O)_{b}H
wherein the molecular weight of the hydrophobe (C₃H₆O) is between approximately 5200 to 9000 and the percentage of hydrophile (C₂H₄O) is between approximately 3% and 15% by weight, whereby a copolymer in accordance with the above formula having a molecular weight of the hydrophobe of approximately 5200 and having the hydrophile percentage of the total molecular weight of 5% is excluded.

2. The surface-active copolymer of claim 1 having the following formula:
HO(C₂H₄O)_{b}(C₃H₆O)ₐ(C₂H₄O)_{b}H
wherein the molecular weight of the hydrophobe (C₃H₆O) is approximately 8600 and the percentage of hydrophile (C₂H₄O) is approximately 5% by weight.

3. Use of the surface-active copolymer according to any of claims 1 or 2 for the preparation of a vaccine composition.

4. Vaccine composition comprising the surface-active copolymer according to any of claims 1 or 2.

5. The vaccine composition of claim 4, further comprising
a. oil
b. a non-ionic surface active agent suitable for forming water-in-oil emulsions and
c. silica
wherein the surface-active copolymer, the oil, the non-ionic surface active agent suitable for forming water-in-oil emulsions and the silica are admixed together.

6. The vaccine composition of claim 5, wherein the oil is an animal oil.

7. The vaccine composition of claim 6, wherein the animal oil is squalane.

8. The vaccine composition of claim 5, wherein the non-ionic surface active agent is sorbitan monooleate.

## Patentansprüche

1. Oberflächenaktives Copolymer zur Verwendung in einem Impfstoff mit der folgenden Formel
HO(C₂H₄O)_{b}(C₃H₆O)ₐ(C₂H₄O)_{b}H
worin das Molekulargewicht des hydrophoben (C₃H₆O) zwischen ungefähr 5200 und 9000 liegt und der Prozentanteil des hydrophilen (C₂H₄O) zwischen ungefähr 3% und 15% Gewichtsprozent liegt, wobei ein Copolymer entsprechend der obigen Formel mit einem Molekulargewicht des hydrophoben Anteils von ungefähr 5200 und einem Prozentsatz des hydrophilen Anteils an dem Gesamtmolekulargewicht von 5% ausgenommen ist.

2. Oberflächenaktives Copolymer nach Anspruch 1 mit der folgenden Formel:
HO(C₂H₄O)_{b}(C₃H₆O)ₐ(C₂H₄O)_{b}H
worin das Molekulargewicht des hydrophoben (C₃H₆O) ungefähr 8600 beträgt und der Prozentsatz des hydrophilen (C₂H₄O) ungefähr 5 Gewichtsprozent ausmacht.

3. Verwendung des oberflächenaktiven Copolymers nach einem der Ansprüche 1 oder 2 zur Herstellung einer Impfstoffzusammensetzung.

4. Impfstoffzusammensetzung, die das oberflächenaktive Copolymer nach einem der Ansprüche 1 oder 2 enthält.

5. Impfstoffzusammensetzung nach Anspruch 4, die zusätzlich
a. Öl
b. ein nichtionisches oberflächenaktives Mittel, das geeignet ist zur Ausbildung von Wasser-in-Öl-Emulsionen und
c. Siliciumdioxid
enthält,
wobei das oberflächenaktive Copolymer, das Öl, das nichtionische oberflächenaktive Mittel, das geeignet ist zur Bildung von Wasser-in-Öl-Emulsionen, und das Siliciumdioxid miteinander vermischt sind.

6. Impfstoffzusammensetzung nach Anspruch 5, wobei das Öl ein tierisches Öl ist.

7. Impfstoffzusammensetzung nach Anspruch 6, wobei das tierische Öl Squalan ist.

8. Impfstoffzusammensetzung nach Anspruch 5, wobei das nichtionische oberflächenaktive Mittel Sorbitanmonooleat ist.

## Revendications

1. Un copolymère à surface active, pour l'utilisation dans un vaccin, de formule suivante :
HO(C₂H₄O)_{b}(C₃H₆O)ₐ(C₂H₄O)_{b}H,
dans laquelle le poids moléculaire du (C₃H₆O) hydrophobe est approximativement entre 5 200 à 9 000 et le pourcentage du (C₂H₄O) hydrophile est approximativement entre 3 % et 15% en poids, parmi lequel un copolymère en accord avec la formule ci-dessus ayant un poids moléculaire de l'hydrophobe approximativement de 5 200 et ayant un pourcentage de l'hydrophyle du poids moléculaire total de 5 % est exclu.

2. Le copolymère à surface active selon la revendication 1, de formule suivante :
HO(C₂H₄O)_{b}(C₃H₆O)ₐ(C₂H₄O)_{b}H,
dans laquelle le poids moléculaire du (C₃H₆O) hydrophobe est approximativement 8 600 et le pourcentage du (C₂H₄O) hydrophile est approximativement 5 % en poids.

3. Utilisation du copolymère à surface active selon l'une quelconque des revendications 1 ou 2 pour la préparation d'une composition de vaccin.

4. Composition de vaccin comprenant le copolymère à surface active selon l'une quelconque des revendications 1 ou 2.

5. La composition de vaccin selon la revendication 4, comprenant de plus :
a. de l'huile
b. un agent à surface active utile pour des émulsions eau dans l'huile et
c. de la silice.

6. La composition de vaccin selon la revendication 5, dans laquelle l'huile est une huile animale.

7. La composition de vaccin selon la revendication 6, dans laquelle l'huile animale est le squalane.

8. La composition de vaccin selon la revendication 5, dans laquelle l'agent à surface active non ionique est le monoléate de sorbitane.
